Europäisches Patentamt

European Patent Office

Office européen des brevets

(11) Publication number: **0 264 017 B1**

# EUROPEAN PATENT SPECIFICATION

(45) Date of publication of patent specification: **25.09.91**   (51) Int. Cl.⁵: **A61K 37/50**

(21) Application number: **87114135.4**

(22) Date of filing: **28.09.87**

(54) **Pharmaceutical compositions having healing activity.**

(30) Priority: **17.10.86 IT 2205186**

(43) Date of publication of application:
**20.04.88 Bulletin 88/16**

(45) Publication of the grant of the patent:
**25.09.91 Bulletin 91/39**

(84) Designated Contracting States:
**AT BE CH DE ES FR GB GR IT LI LU NL SE**

(56) References cited:

**No relevant documents have been disclosed**

(73) Proprietor: **BIOINDUSTRIA FARMACEUTICI S.p.A.**
**Via De Ambrosiis, 2-4-6**
**I-15067 Novi Ligure Alessandria(IT)**

(72) Inventor: **Federici, Giorgio**
**Via De Ambrosiis, 2-4-6**
**I-15067 Novi Ligure Alessandria(IT)**
Inventor: **Bertoncelli, Maria Luisa**
**Via De Ambrosiis, 2-4-6**
**I-15067 Novi Ligure Alessandria(IT)**

(74) Representative: **Minoja, Fabrizio**
**Studio Consulenza Brevettuale Via Rossini, 8**
**I-20122 Milano(IT)**

## Description

The present invention concerns pharmaceutical compositions having healing activity, containing cytochrome-c-peroxidase (ferrocytochrome c: hydrogen peroxide oxidoreductase) as an active principle.

It is known that, following tissue damage such as wounds and burns, hydrogen peroxide formation occurs at the damaged site level, as a consequence of the concomitant inflammatory process.

The presence of hydrogen peroxide impairs the healing process.

Pharmaceutical compositions having healing activity, containing the enzyme catalase as active principle, are also known.

However, catalase has a reduced affinity towards its substrate, as it is evident from the Km values reported in the literature:

cytochrome-c-peroxidase     $Km\ H_2O_2 = 4.5 \times 10^{-6}$ M

catalase     $Km\ H_2O_2 = 1.1$ M

Said reduced affinity of catalase involves a slow reaction rate of the enzyme itself, with a consequent $H_2O_2$ accumulation.

Moreover, the pharmaceutical compositions containing catalase have the drawback of a low stability, because of the instability of the enzyme present.

It has now been found that the cytochrome-c-peroxidase, hereinafter defined also with the abbreviation CCP, may be advantageously used as an healing agent.

The CCP is highly specific for ferrocytochrome c, catalyzing the peroxidase oxidation thereof ($H_2O_2 + 2$ ferrocytochrome $\rightarrow$ 2 ferrocytochrome + $2H_2O$).

With respect to catalase, the cytochrome-c-peroxidase has the advantage of an higher affinity towards $H_2O_2$:

$Km\ H_2O_2 = 4.5 \times 10^{-6}$ M

$Km\ citc.Fe^{2+} = 1 \times 10^{-5}$ M

and hence the ability of reacting at the maximum rate even at low concentrations of $H_2O_2$, preventing thereby an accumulation thereof, thanks to a constant and immediate removal, allowing therefore a faster healing process.

Moreover, the cytochrome-c-peroxidase is remarkably more stable than catalase, with consequent handling and storage advantages.

The cytochrome-c-peroxidase present in the pharmaceutical compositions of the invention is prepared according to a process comprising the lysis of commercial brewer's yeast, in the presence of ethyl acetate, aqueous extraction and subsequent purification by chromatography on diethylaminoethyl cellulose.

The crude product obtained is concentrated by ultrafiltration and purified by gel filtration on BIOGEL P-100 in sodium acetate 0.1 M pH = 5.0 buffer.

The fractions showing an absorption ratio 408/280 equal or higher than 1.1, are collected, dialyzed and lyophilized.

The so-prepared cytochrome-c-peroxidase is an amorphous, hygroscopic powder, yellow-rose in colour and completely soluble in water. The molecular weight, determined by gel filtration, is 40.000 daltons and the enzyme, at the final purification step, shows a single band on polyacrylamide gel. The specific activity is from 570 to 618 U/mg (unit = amount of enzyme able to oxydize 1 micromole of horse ferrocytochrome-c per minute in the standard conditions of activity).

The optimal temperature is 23-25° C.

In solution, the enzyme loses its activity in a few hours at room temperature, at 0° C it is stable for more than 2 months and at -20° C it does not show activity loss over a prolonged period.

Heating for 5 hours at 80° C causes a complete activity loss. In the UV spectrum the enzyme has a maximum absorption at 280 nm, in the visible at 408 nm. The A 408/A280 is an important index for evaluating the enzyme purity; said index increases as soon as the purification reaches a value of 1.24 - 1,4 for the enzyme at the final step.

The enzymatic activity is determined by measuring the ferrocytochrome c oxidation catalyzed by the enzyme, operating at 25° C in a solution containing:

20 mM phosphate buffer pH = 7

170 $\mu$M $H_2O_2$

5 mM reduced cytochrome c.

The final volume is 2 ml.

The reaction is started by the addition of 10 $\mu$l of enzyme solution, suitably diluted. The absorption decrease at 550 nm is proportional to the percentage of oxidized ferrocytochrome and to the enzyme concentration. An $\epsilon$ 27.6 nM$^{-1}$ cm$^{-1}$ and a $\Delta$ (reduced cytochrome minus oxidized cytochrome) of 19.6

2

$mM^{-1}$ $cm^{-1}$ were used.

The cytochrome-c-peroxidase having the reported chemical and enzymatic characteristics has been subjected to some experimental tests in order to evaluate whether the products exerts an influence on the healing and repairing process of epithelial lesions.

## 1. In vitro study: CCP action on the proliferative activity of human cutaneous fibroblasts.

10 flasks containing a monolayer of human cutaneous fibroblasts in 6% MEM with added fetal calf serum were used. The flasks were divided as follows:

I batch (5 flasks): controls

II batch (5 flasks): CCP treated

In the control cells no kind of additive, different than those already contained, was added.

The flasks were observed 12, 24, 48 and 72 hours after the CCP addition, by means of an inversion microscope.

The obtained results showed, in the CCP treated cells, an increase of cellularity as well as a significant increase of blastization and a multi-layered arrangement.

In the control fibroblasts the cellular activity turned out to be low, no blastization process was noticed ant the cells are arranged in a monolayer.

In order to confirm these data, the count of fibroblasts of the two groups under study has been carried out. The cell number in the treated group was significantly higher than the controls.

## 2. In vivo study

### a) Healing activity on the cutaneous lesion in the normal rat

Male Sprague-Dawley rats, weighing 200 g, housed in standard conditions, were used. The animals were divided in two groups (10 animals each) and treated 4 times a day for five days:

I group: controls - lanoline + vaseline

II group: treated with CCP 0.4% in plastonite.

Bilateral lesions 5 mm in diameter were produced on the back in each animals by means of a suitable apparatus, said lesions crossing the whole thickness of the epidermal layers up to the subcutaneous one. At the end of treatment, the lesions were removed by a lozenge dissection and kept in formaline for the microscopic examination (hematoxylin-eosin), in order to observe the possible presence of granulation tissue and possible re-epitelization signs.

The obtained results showed, for the CCP treated rats, that the aspect of the bottom is granulated and that there is no presence of exudate. In the control rats the aspect of the bottom is, on the contrary, irregular and shows presence of exudate. At the microscopic examination, in the treated animals, an hypercellularity is noticed, with presence of granulation tissue and fibroblasts and appearance of neo-vascularization phenomenon; only in some treated animals a slight degree of re-epitalization in some areas of the lesion bottom is noticed.

### b) Healing activity on the cutaneons lesion in the diabetic rat

Male albino Wistar rats, mean weight 350 g, housed in standard conditions, were treated with a single dose of streptozotocine (60 mg/kg, i.p.). Said dose is sufficient to cause a total and permanent destruction of the pancreatic insulin-secreting cells. The cutaneous lesions were provoked, after suitable peeling, on the cutis of the back of diabetic rats by a dermotome, up to a depth of about 5 mm. CCP was applied to a group of rats every 12 hours, in form of 2% ointment in plastonite.

The control animals were similarly treated with the ointment containing only plastonite. The healing process was followed with observations before of each application of the two kinds of ointment.

The obtained results, reported in the following table I, show a faster healing after application of the CCP ointment, in comparison to the animals treated with the vehicle alone.

TABLE 1

| Effects of CCP product on the cutaneons lesions of diabetic rat | | |
|---|---|---|
| HOURS SINCE THE CUTANEOUS LESION | DIABETIC RATS Controls Treated | |
| | (vehicle alone) (n = 12) | (CCP) (n = 12) |
| 0 | 0.0 | 0.0 |
| 12 | 0.0 | 0.0 |
| 24 | 0.0 | 0.0 |
| 36 | 0.0 | 16.6 |
| 48 | 0.0 | 25.0* |
| 60 | 0.0 | 41.6* |
| 72 | 16.6 | 50.0* |
| 84 | 25.0 | 75.0* |
| 96 | 25.0 | 83.3* |
| 108 | 50.0 | 100.0* |
| 120 | 75.0 | 100.0 |
| 132 | 100.0 | 100.0 |
| The values are expressed as percentage (number of repaired lesions against number of performed lesions). | | |

*) Significant difference in comparison with the control (p < 0.005, Fisher exact T-test)

## c) Healing activity on the superficial lesion of corneal epithelium in the rabbit.

Male albino rabbits of the New Zealand strain, mean weight of 1.8 kg, housed in standard conditions, were used.

The corneal lesions were provoked on both eyes of 12 rabbits by scarification with a glass cylinder 3 mm in diameter. The lesions were made on the optical zone of the cornea, at a depth of 2-3 mm to save the Descemet membrane. The ointment containing CCP (2%) has been applied on the right eye of each rabbit every 12 hours from the lesion. The ointment containing only plastonite was applied on the left eye with the same procedures. The rabbits eyes were observed under a slit-lamp immediately after the scarification and before of each ointment application.

The obtained results were reported in the following Table 2.

It is noticed that the CCP ointment caused a repairing of the corneal lesions faster than that observed in the control animals treated with the vehicle alone (Table 2). Infact, all the treated eyes showed a total repair of the corneal lesions already 48 hours after the scarification. The control eyes showed a total repair of the corneal lesion only 72 hours after scarification.

TABLE 2

| Effect of CCP on the rabbit corneal lesions. | | |
|---|---|---|
| HOURS SINCE THE CUTANEOUS | DIABETIC RATS Controls Treated | |
| | (vehicle alone) (n = 12) | (CCP) (n = 12) |
| 0 | 0.0 | 0.0 |
| 12 | 0.0 | 0.0 |
| 24 | 0.0 | 16.6 |
| 36 | 25.0 | 50.0 |
| 48 | 41.6 | 100.0* |
| 60 | 50.0 | 100.0* |
| 72 | 100.0 | 100.0 |
| The values are expressed as percentage (number of eyes with repaired lesions against number of treated rats). | | |

*) Significant difference over the controls ($p < 0.05$, Fisher exact test).

The results above reported clearly show that the CCP products has stimulatory effects on the repairing processes of the superficial epithelial lesions, exerting an increase of the cellular activity and inducing a fast re-epitelization.

The CCP may be therefore used as active principles of pharmaceutical compositions having healing activity for topical, ophthalmic or aerosol administration. Examples of suitable formulations comprise, inter alia, ointments, creams, gauze, ophthalmic ointments, eye-drops (collyrium), aerosol.

Some examples of pharmaceutical formulations according to the invention are reported hereinbelow.

The weight percentage of the active principle in the different forms ranges from 0.2 to 1%.

## EXAMPLE 1
## Cutaneous ointment

| Cytochrome-c-peroxidase | | 0.4 g |
|---|---|---|
| Plastonite | q.s.to | 100 g |

The active principle finely pulverized is incorporated into the excipient at room temperature.

## EXAMPLE 2
## Cutaneous ointment

| Cytochrome-c-peroxidase | | 0.2 g |
|---|---|---|
| Ferric chloride | | 4 mg |
| Mannitol | | 5 g |
| Plastonite | q. s. to | 100 g |

## EXAMPLE 3
## Cutaneous ointment

| Cytochrome-c-peroxidase | | 0.5 g |
|---|---|---|
| Vaseline oil | | 25 g |
| Lanoline | | 20 g |
| White vaseline | q. s. to | 100 g |

Cytochrome-c-peroxidase finely dispersed in vaseline oil is added at room temperature to the mixture comprising white vaseline and lanoline.

**EXAMPLE 4**
**Aerosol for pulverulent nebulization**

| Cytochrome-c-peroxidase | | 0.4 g |
|---|---|---|
| Magnesium stearate | | 10 g |
| Lactose | q. s. to | 100 g |

The propellant is a dichloro difluoromethane and tetrafluorodichloroethane mixture in 40/60 ratio.

**EXAMPLE 5**
**Ophthalmic ointment**

| Cytochrome-c-peroxidase | 0.5 g |
|---|---|
| Cetyl alcohol | 4 g |
| Wool wax | 10 g |
| White vaseline | 86 g |
| Vaseline oil | 43 ml |

Cetyl alcohol, wax, white vaseline are dissolved at 70°C in water bath, the mixture is cooled under stirring, finely pulverized cytochrome-c-peroxidase suspended in vaseline oil is added.

**EXAMPLE 6**
**Ophthalmic ointment**

| Cytochrome-c-peroxidase | | 0.2 g |
|---|---|---|
| Benzalkonium chloride | | 0.05 g |
| White Lanoline | | 3 g |
| Vaseline oil | | 10 g |
| White vaseline | q. s. to | 100 g |

**EXAMPLE 7**
**Ophthalmic ointment**

| Cytochrome-c-peroxidase | | 2 g |
|---|---|---|
| Plastonite | q. s. to | 100 g |

**Claims**
**Claims for the following Contracting States : BE, CH, DE, FR, GB, IT, LI, LU, NL, SE, AT, SP, GR**

6

1. Pharmaceutical compositions having healing activity, characterized by containing cytochrome-c-peroxidase as an active principle.

2. Pharmaceutical compositions according to claim 1, for topical, ophthalmic or aerosol administration.

3. Pharmaceutical compositions according to claim 1 or 2 in form of ointment, cream, gauze, ophthalmic ointment, collyrium and aerosol.

**Revendications**

**Revendications pour les Etats contractants suivants : BE, CH, DE, FR, GB, IT, LI, LU, NL, SE, AT, SP, GR**

1. Compositions pharmaceutiques ayant une activité curative, caractérisées par le fait qu'elles contiennent de la cytochrome-c-peroxydase comme principe actif.

2. Compositions pharmaceutiques selon la revendication 1, pour l'administration topique, ophtalmique ou sous la forme d'aérosol.

3. Compositions pharmaceutiques selon la revendication 1 ou 2 se présentant sous la forme d'une pommade, d'une crème, d'une gaze, d'une pommade ophtalmologique, d'un collyre et d'un aérosol.

**Patentansprüche**

**Patentansprüche für folgende Vertragsstaaten : BE, CH, DE, FR, GB, IT, LI, LU, NL, SE, AT, ES, GR**

1. Pharmazeutisches Präparat mit heilender Aktivität, dadurch **gekennzeichnet,** daß es als aktives Prinzip Cytochrom-c-peroxidase enthält.

2. Pharmazeutisches Präparat nach Anspruch 1 für die topische, ophthalmologische Anwendung oder für die Aerosol-Anwendung.

3. Pharmazeutisches Präparat nach Anspruch 1 oder 2 in Form einer Salbe, einer Creme, eines Verbandmulls, einer Augensalbe, eines Augenwassers und eines Aerosols.